# FASCICULE DE BREVET EUROPEEN

(11) **EP 0 611 560 B1**
(45) Date de publication et mention de la délivrance du brevet: **25.09.1996**
(21) Numéro de dépôt: 94400246.8
(22) Date de dépôt: 04.02.1994
(51) Int. Cl.: A61F 2/42

(54) **Elément prothétique pour petites articulations**
Prothesenelement für kleine Gelenke
Prosthetic element for small joints

(30) Priorité: 16.02.1993 FR 9301820
(43) Date de publication de la demande: 24.08.1994
(73) Titulaire: LANDANGER- LANDOS, Société Anonyme, F-52000 Chaumont (FR); Condamine, Jean-Luc, F-14300 Caen (FR)
(72) Inventeur: Condamine, Jean-Luc, F-14300 Caen (FR); Fresard, Alain, F-74240 Gaillard (FR); Moreau, Jean-Charles, F-52000 Chaumont (FR)
(74) Mandataire: Lapoux, Roland

(56) Documents cités:
- WO-A-91/04718
- FR-A- 2 605 878
- FR-A- 2 610 822
- FR-A- 2 617 706
- GB-A- 2 056 862
- US-A- 4 304 011
- US-A- 4 352 212

## Description

La présente invention concerne un élément prothétique pour petites articulations, notamment articulations métacarpo-phalangiennes et interphalangiennes proximales de la main.

Une maladie rhumatoïde siégeant dans les articulations métacarpo-phalangiennes ou interphalangiennes proximales, ou une fracture de ces articulations, entraînent une destruction des structures articulaires et une déformation de ces articulations. La mobilité de la main et la fonction de préhension sont compromises. Pour restaurer celles-ci, une résection suivie d'une implantation de prothèse est pratiquée.

Les prothèses proposées jusqu'ici pour ces petites articulations peuvent être classées en des premier et second groupes : les prothèses du premier groupe sont en fait des "espaceurs" flexibles au sein desquels s'effectue le mouvement; les prothèses du second groupe sont réalisées en deux éléments stabilisés l'un par rapport à l'autre avec ou sans moyen de liaison assurant leur mouvement relatif.

Les prothèses du premier groupe, telles que l'implant articulaire du Docteur Alfred B. SWANSON commercialisé sous la marque "SILASTIC" par la société DOW CORNING WRIGHT, Arlington, US, sont monoblocs et réalisées en un matériau flexible, tel qu'élastomère de silicone. Elles comportent trois parties : deux tiges coniques implantées dans les diaphyses osseuses et une liaison centrale souple reliant les tiges, dont la flexibilité autorise la mobilisation de l'articulation et dont la forme contribue au maintien de l'espace de résection articulaire. Le remplacement articulaire réalisé par cet implant n'est pas véritablement une arthroplastie mais une résection des surfaces articulaires dont le vide est comblé par l'implant.

Les inconvénients relatés avec ce type d'implant consistent en une détérioration du matériau, un résultat fonctionnel peu satisfaisant et une récidive fréquente des déviations axiales. La détérioration du matériau, tel qu'élastomère de silicone, aboutit à sa fragmentation, qui induit des réactions locales à des corps étrangers suffisamment importantes pour obliger à l'ablation du matériel prothétique. Le résultat fonctionnel est souvent limité en l'absence d'une liaison stable entre les membres de l'articulation qui se traduit par une force de préhension faible.

Les prothèses du second groupe répondent aux critiques des prothèses du premier groupe, et sont composées de deux éléments distincts réalisés en des matériaux plus rigides. Selon un premier type (GB-A-2 056 862 et US-A-4 352 212), les éléments sont reliés ensemble par un axe de charnière n'assurant une mobilité articulaire que dans un plan de l'espace et étant ainsi sujet à des contraintes en cisaillement engendrant des descellements et fractures prothétiques. Selon un second type (GB-A-2 126 097 ; FR-A-2 605 878), les éléments présentent deux surfaces congruentes en contact l'une avec l'autre et glissant l'une par rapport à l'autre lors des mouvements de l'articulation. Ce glissement remédie aux risques de rupture des charnières dans les prothèses du premier type.

Avant son implantation, une prothèse doit être choisie pour être adaptée à la morphologie du patient. Cette adaptation notamment dimensionnelle, affinée au cours de l'intervention, est effectuée par le choix d'une taille de prothèse parmi un petit nombre de tailles proposées, typiquement entre cinq et dix. Ce choix limité implique bien souvent une inadaptation au moins de l'une des tiges prothétiques ou du moyen de liaison entre celles-ci aux caractéristiques anatomo-physiologiques de l'articulation à remplacer, ce qui peut compromettre le résultat opératoire attendu.

Des prothèses métacarpo-phalangiennes autorisant une bonne adaptation anatomo-physiologique ont été proposées (US-A-4 304 011, US-A-4 352 212 et WO-A-9 104 718). Ces prothèses sont du second groupe et comprennent un élément proximal et un élément distal. Chaque élément distal ou proximal est constitué d'une tête et d'une queue intra-diaphysaire. La tête comprend une surface de contact concave ou convexe ainsi qu'une tige saillant à l'arrière de la surface de contact et propre à être insérée dans un trou ménagé dans la queue intra-diaphysaire. La forme de la tête doit être adaptée d'une part au contact avec la tête de l'autre élément distal ou proximal et d'autre part en alignement avec la queue intra-diaphysaire pour former avec celle-ci un ensemble rigide. En conséquence, la fabrication de la tête est délicate et complexe et rend l'élément prothétique coûteux.

La présente invention vise à fournir un élément prothétique du second groupe pour petites articulations, notamment articulations métacarpo-phalangiennes et interphalangiennes proximales, autorisant une bonne adaptation anatomo-physiologique, offrant un coût de fabrication plus faible, tout en étant quasi-monolithique après implantation.

A cette fin, un élément prothétique pour petites articulations comprenant une tête, une queue intra-diaphysaire qui sont deux pièces distinctes et un moyen de stabilisation pour stabiliser en rotation la tête par rapport à la queue intra-diaphysaire à laquelle elle est liée, est caractérisé en ce qu'il comprend une tige de liaison sensiblement cylindrique s'étendant entièrement dans la tête et la queue intra-diaphysaire et ayant des extrémités qui sont liées rigidement de manière démontable à la tête et la queue dans des trous ménagés dans la tête et la queue intra-diaphysaire.

Un élément prothétique comprend ainsi trois pièces qui sont chacune de fabrication relativement peu onéreuse. Après implantation, l'élément prothétique est quasi-monolithique grâce à la double liaison de la tête et de la queue intra-diaphysaire par la tige de liaison et le moyen de stabilisation.

Une prothèse pour petites articulations comprend alors des premier et second éléments prothétiques selon l'invention. Le premier élément présente une surface convexe dont une partie est complémentaire de et en contact avec une surface concave de la tête du second élément.

Il est ainsi possible de choisir la taille de la queue indépendamment de la taille de la tête dans un élément, d'une part, et la taille de la queue du premier élément indépendamment de la taille de la queue du second élément d'autre part. L'adaptation anatomo-physiologique est assurée par le nombre élevé de combinaisons possibles.

Pour une bonne adaptation anatomique, les surfaces externes des têtes sont sensiblement semi-sphériques et les têtes sont respectivement décentrées par rapport aux axes longitudinaux des queues sensiblement alignées longitudinalement. La tête du second élément comprend une butée dorsale pour empêcher une sub-luxation antérieure et deux butées latérales pour guidage latéral.

Selon une autre caractéristique de l'invention, le moyen de stabilisation dans chacun des premier et second éléments comprend un assemblage à tenon et rainure complémentaires.

Les queues intra-diaphysaires sont en matière plastique et les tiges de liaison sont métalliques. Les tiges de liaison rigidifient les queues intra-diaphysaires.

Après implantation, le mouvement articulatoire est assuré par le glissement des têtes l'une par rapport à l'autre. Les têtes ayant des surfaces congruentes sont respectivement métallique et en matière plastique, telle que polyéthylène, ou toutes deux en métal. Le glissement peut donc être de type métal/matière plastique, ou métal/métal.

D'autres caractéristiques et avantages de la présente invention apparaîtront plus clairement à la lecture de la description suivante de plusieurs réalisations préférées de l'invention, en référence aux dessins annexés correspondants dans lesquels :
- la figure 1 est une vue en coupe longitudinale antéro-postérieure et palmaire-dorsale prise le long de la ligne I-I de la figure 2 d'une prothèse selon l'invention pour articulation métacarpo-phalangienne;
- la figure 2 est une vue dorsale en coupe longitudinale antéro-postérieure et latérale prise le long de la ligne II-II de la figure 1 ;
- la figure 3 une vue longitudinale de côté d'une queue prothétique intra-diaphysaire ;
- la figure 4 est une vue en bout, côté tête, de la queue intra-diaphysaire ;
- les figures 5 et 6 sont deux vues en coupe longitudinale, respectivement analogues aux figures 1 et 2 et prises le long de la ligne V-V de la figure 6 et de la ligne VI-VI de la figure 5, d'une tête articulaire à surface convexe ;
- la figure 7 est une vue longitudinale d'une première réalisation d'une tige interne de liaison ;
- la figure 8 est une vue longitudinale d'une seconde réalisation d'une tige interne de liaison ; et
- les figures 9 et 10 sont deux vues en coupe longitudinale, respectivement analogues aux figures 1 et 2 et prises le long de la ligne IX-IX de la figure 10 et de la ligne X-X de la figure 9, d'une tête articulaire à surface concave.

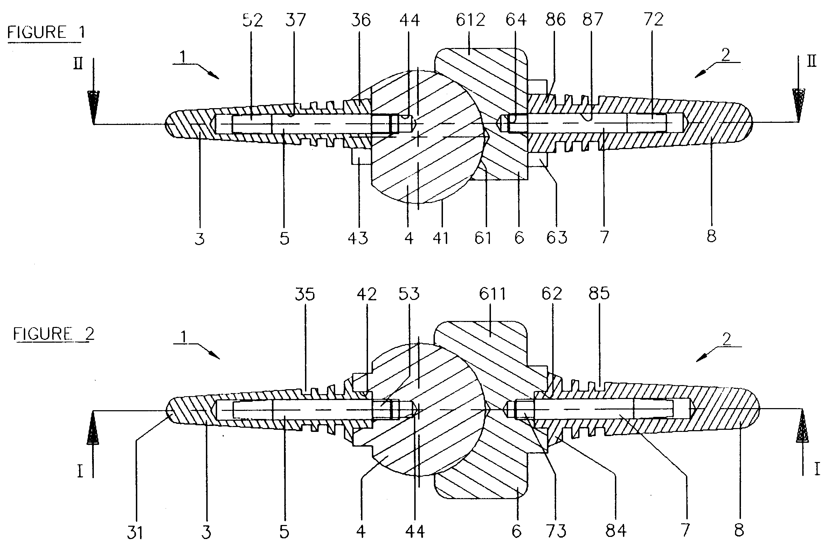

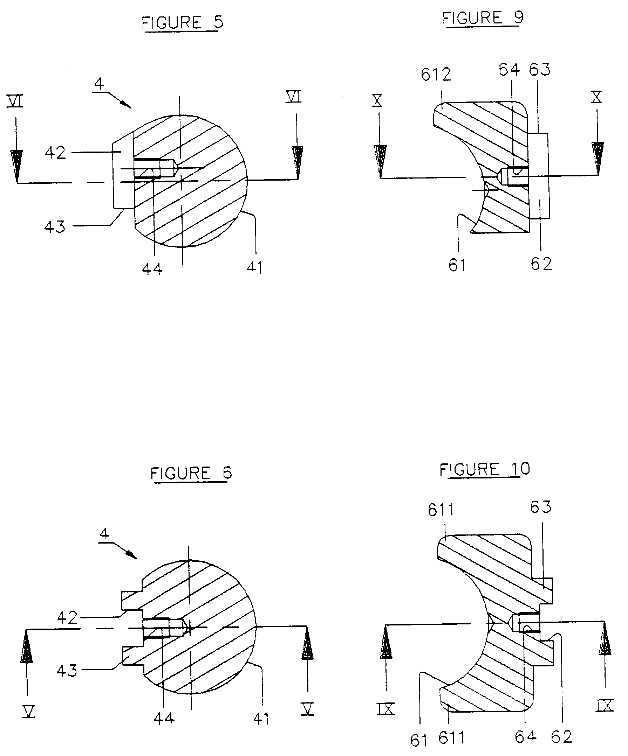

Une prothèse selon l'invention comprend un premier élément 1, dit élément proximal et un second élément 2, dit élément distal. La prothèse illustrée aux figures 1 et 2 concerne, à titre d'exemple, une prothèse pour articulation métacarpo-phalangienne, l'élément 1 étant à implanter dans le métacarpe et l'élément 2 étant à implanter dans la première phalange. Dans la figure 1, les régions dorsale et palmaire sont respectivement en parties supérieure et inférieure.

L'élément 1 comprend trois pièces séparables assemblées rigidement, à savoir une queue intra-diaphysaire 3, une tête articulaire 4 et une tige interne de liaison 5.

La queue intra-diaphysaire 3 est réalisée en un matériau rigide implantable, tel que polyéthylène. Comme représenté aux figures 1 à 4, la queue 3 est symétrique par rapport à deux plans longitudinaux perpendiculaires ayant pour traces les lignes II-II et I-I dans les figures 1 et 2 respectivement.

La queue 3 a une surface tronconique externe ayant des sections ovales et des génératrices sensiblement concaves, et une extrémité arrondie 31.

La surface tronconique présente deux tronçons. Le premier tronçon 32 est lisse et s'étend depuis l'extrémité 31 jusqu'au second tronçon 33. Ce second tronçon présente des collerettes 34 constituant entr'elles des gorges 35, formant anfractuosités, régulièrement réparties longitudinalement. Le tronçon 33 s'étend jusqu'à la grande base de la queue intra-diaphysaire.

Devant la plus large collerette 34 formant la grande base de la surface tronconique saille un tenon diamétral 36 sensiblement rectangulaire. A partir de la face du tenon 36 est percé un trou cylindrique lisse axial borgne 37 dans la queue 3.

Selon d'autres variantes de réalisation non représentées, la section de la surface tronconique de la queue 3 est circulaire et/ou présente des collerettes et anfractuosités alternées depuis la petite base jusqu'à la grande base.

En référence aux figures 5 et 6, la tête articulaire 4 de l'élément proximal présente une surface sphérique convexe 41 dans une calotte de laquelle a été usinée une chape constituée par une rainure 42 et deux branches droites parallèles 43 bordant la rainure. La rainure 42 est complémentaire au tenon de queue 36. Un trou taraudé borgne 44 est pratiqué perpendiculairement depuis le fond de la rainure 42. L'axe du trou 44 est situé dans le plan diamétral à la surface sphérique 41 ayant pour trace I-I dans la figure 2 et V-V dans la figure 6 et constituant un plan de symétrie longitudinal à la rainure 42 et à la tête proximale 4. Le diamètre du trou 44 est sensiblement identique à celui du trou 37 de la queue intra-diaphysaire. La surface sphérique 41 est décentrée par rapport à l'axe du trou 44, comme cela est visible aux figures 1 et 5. En variante, le trou 44 est lisse.

La tige interne de liaison 5 est une tige cylindrique métallique dont la surface au moins en partie centrale 51 destinée à pénétrer sensiblement dans la première moitié du trou de queue 37 est lisse. Le diamètre de la tige interne de liaison est sensiblement égal à celui des trous 37 et 44.

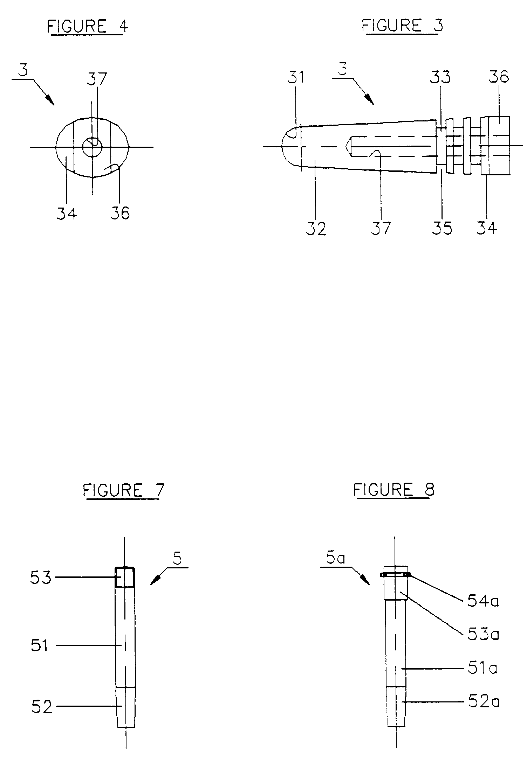

Selon la réalisation illustrée à la figure 7, une première extrémité 52 de la tige 5 est tronconique convergente afin de constituer un cône de pénétration permettant l'assemblage par emmanchement à force de la tige 5 dans le trou de queue 37 ; une seconde extrémité 53 de la tige 5 est cylindrique et filetée pour être vissée dans le trou taraudé 44. En correspondance avec la variante de trou 44 lisse, une seconde réalisation de tige 5a est montrée à la figure 8. Selon cette réalisation, une première extrémité 52a de la tige 5a est tronconique convergente afin de constituer un assemblage à cônes complémentaires mâle et femelle avec un fond tronconique du trou de queue 37; une seconde extrémité 53a de la tige 5a est cylindrique et comporte un petit anneau souple ou fendu 54a cerclant une petite gorge de la tige pour pénétrer à force dans une gorge du trou lisse de tête 44.

Selon d'autres variantes d'assemblage, l'extrémité 52 de la tige 5 est filetée pour être vissée dans le trou au moins partiellement taraudé 37, correspondant dans la queue 3, avant que l'autre extrémité 53a de la tige 5a soit enfilée dans le trou lisse 44 correspondant dans la tête 4, pour constituer avec ce dernier un assemblage par pénétration en translation sans rotation et blocage en translation et rotation, par exemple avec des cônes mâle et femelle complémentaires, un anneau encliquetant dans une gorge, des petites billes rappelées radialement par ressort encliquetant dans des encoches, etc.

Pour constituer l'élément proximal 1, l'extrémité 53 de la tige interne de liaison 5 est engagée dans le trou 44 de la tête articulaire, et la tige de liaison 5 est vissée dans la tête 4 pour les lier rigidement. Selon la variante de réalisation de tige 5a illustrée à la figure 8, l'extrémité 53a de la tige interne de liaison 5a est engagée dans le trou 44 de la tête articulaire, de manière à emmancher à glissement la tige 5a dans la tête 4 pour les lier rigidement au moyen de l'anneau 54a.

La tige de liaison 5 est ensuite emmanchée à force dans le trou 37 de la queue intra-diaphysaire 3 jusqu'à ce que le tenon 36 de la queue vient en butée et s'emboîte dans la rainure 42 de la tête. Les trois pièces de l'élément proximal sont ainsi assemblées rigidement, chacune des pièces 3, 4 et 5 étant immobile en translation et rotation par rapport aux deux autres. En variante, la tige 5a est emmanchée à glissement dans le trou 37 de la queue 3 jusqu'à ce que l'extrémité tronconique 52a de la tige de liaison 5a soit coincée dans un fond tronconique du trou 37. Simultanément, le tenon 36 s'emboîte dans la rainure 42 de la tête.

La tige de liaison 5 rigidifie la queue 3 et lie cette dernière avec la tête articulaire. L'emboîtement du tenon 36 et de la rainure 42 empêche la rotation de la tête par rapport à la queue intra-diaphysaire. Après assemblage de l'élément proximal, la queue 3, la tige de liaison 5 et le trou 44 de la tête 4 sont alignés axialement en coupe horizontale comme montré à la figure 2. En coupe verticale, comme montré à la figure 1, la surface sphérique 41 est décentrée par rapport à l'axe de la tige 5 et de la queue 3. L'élément proximal une fois assemblé est symétrique par rapport au plan longitudinal de trace I-I dans la figure 2.

En référence aux figures 1 et 2, l'élément distal 2 comprend une tête articulaire 6, une tige de liaison 7 et une queue intra-diaphysaire 8.

La tige de liaison 7 et la queue 8 sont analogues respectivement à la tige de liaison 5 et à la queue 3 de l'élément proximal, et sont agencées entr'elles et avec la tête 6, comme les agencements mutuels des pièces d'élément proximal 3, 4 et 5.

En référence aux figures 9 et 10, la tête articulaire 6 est réalisée en une matière plastique telle que polyéthylène. Elle est symétrique par rapport au plan longitudinal de trace IX-IX dans la figure 10 et I-I dans la figure 2. La tête 6 présente, en face postérieure, une cavité sensiblement semi-sphérique 61 complémentaire de la surface sphérique 41 de la tête proximale afin de reconstituer la fonction de l'articulation métacarpo-phalangienne. Dans un plan en coupe axiale horizontale, comme montré aux figures 2 et 10, la cavité 61 est limitée par des bordures symétriques 611 alignées suivant une corde en retrait du centre de la surface sphérique. Dans un plan en coupe verticale, palmaire-dorsale, comme montré aux figures 1 et 9, la cavité 61 est bordée par une butée supérieure dorsale 612 alignée avec le plan d'alignement des bordures 611. En partie inférieure et palmaire, la cavité 61 est ouverte, quasiment à mi-hauteur depuis le plan diamétral horizontal de trace II-II dans la figure 1 et X-X dans la figure 9.

La tête 6 comprend également, comme la tête 4, une chape à rainure 62 et bordures 63 verticales, et un trou cylindrique taraudé borgne 64 ménagé au centre de la rainure 62 et dont l'axe est situé dans le plan de symétrie de trace IX-IX. La rainure 62 et le trou 64 sont respectivement analogues à la rainure 42 et au trou 44 de la tête proximale 4. En variante, le trou 64 est lisse.

L'assemblage des trois pièces 6, 7 et 8 en l'élément distal 2 est analogue à celui des pièces d'élément proximal 3, 4 et 5.

La tige de liaison 7 est vissée dans le trou taraudé 64 de la tête 6 et emmanchée à force dans le trou 87 de la queue intra-diaphysaire 8, de manière à ce que la plus large collerette 84 de la queue 8 vienne en butée contre les bordures de rainure 63 de la tête 6 et le tenon 86 de la queue 8 s'emboîte dans la rainure 62 de la tête 6. Le blocage en translation et rotation est également obtenu au moyen d'un assemblage de type emmanchement à force entre la tige de liaison 7 et la queue 8, et d'un assemblage par vissage entre la tige 7 et la queue 8. En variante, la tige de liaison 7 est emmanchée à force dans le trou 64 qui est lisse. Selon une autre variante, une extrémité postérieure de la tige 7 comprend un cylindre pourvu d'un anneau de blocage qui s'assemble avec un trou lisse à gorge 64 de la tête 6. Selon encore une autre variante, la tige 7 et la queue 8 sont assemblées par cônes complémentaires mâle et femelle. Tout autre moyen d'assemblage rapide connu tels que ceux décrits précédemment peut être utilisé.

La prothèse est implantée en introduisant les queues 3 et 8 respectivement dans des fûts diaphysaires de segment osseux métacarpien et phalangien exposés après la résection des surfaces articulaires. Les anfractuosités 35 et 85 sur les surfaces irrégulières des queues assurent un ancrage osseux direct sans interposition de ciment. La section transversale ovale des queues intra-diaphysaires empêche toute rotation des queues intra-diaphysaires par rapport au milieu osseux.

Après implantation et sélection des pièces et assemblage des éléments proximal et distal de la prothèse, les surfaces sphériques congruentes 41 et 61 respectivement convexe et concave sont en contact, comme montré aux figures 1 et 2. Ce contact est maintenu par le réglage per-opératoire de la tension des structures péri-articulaires, et par le rapport entre la taille des éléments implantés et celle des résections osseuses.

La fonction articulaire de la prothèse se traduit par un glissement métal/polyéthylène de la surface convexe 41 sur la surface concave 61.

La butée dorsale 612 de la tête distale empêche une sub-luxation antérieure, c'est-à-dire le passage de la surface articulaire distale 61 sous la tête proximale 4.

Les surfaces articulaires sphériques 41 et 61 telles que représentées sont plus particulièrement destinées à une arthroplastie métacarpo-phalangienne, fournissant ainsi une prothèse à deux degrés de liberté, permettant une mobilité antéro-postérieure et latérale.

Selon une variante de réalisation non représentée, les surfaces articulaires congruentes 41 et 61 ne sont pas simplement sphériques, mais présentent en outre l'une une gorge ou sillon sensiblement circulaire et l'autre une crête sensiblement circulaire, cette gorge et cette crête étant sensiblement complémentaires et glissant l'une sur l'autre.

La surface convexe 41 comprend par exemple une crête sensiblement circulaire située dans le plan de symétrie montré à la figure 1, ayant pour trace I-I dans la figure 2, et la surface concave comprend une gorge complémentaire à la crête, également sensiblement circulaire et situé dans le plan de symétrie. Ce type de surface articulaire convient plus particulièrement à une arthroplastie interphalangienne proximale, fournissant ainsi une prothèse à un seul degré de liberté, stabilisée en direction antéro-postérieure et direction latérale.

La réalisation en trois pièces séparables de chaque élément de prothèse permet au praticien de choisir entre plusieurs types de tête, différents par leurs surfaces articulaires, notamment pour une prothèse métacarpo-phalangienne ou pour une prothèse interphalangienne proximale.

De plus, pour un type de tête donné, la taille de la tête est choisie en fonction de l'anatomo-physiologie du patient, par exemple parmi trois tailles.

En outre, la taille de chacune des queues intra-diaphysaires est choisie, indépendamment l'une de l'autre et indépendamment de la tête, en fonction de la morphologie du patient, par exemple parmi huit tailles.

Ainsi, par exemple avec un jeu de trois tailles de tête et deux jeux de chacun huit tailles de queue intra-diaphysaire, soit au total 3 + 8 + 8 = 19 pièces, le chirurgien constitue la prothèse à implanter en sélectionnant une taille de tête parmi trois et deux tailles de queues parmi huit. Il a donc 3 x 8 x 8 = 192 prothèses réalisables à sa disposition, ce qui permet une adaptation fine aux caractéristiques anatomo-physiologiques de l'articulation du patient à remplacer.

Cette adaptation est économique, puisque le fabricant de prothèse ne livre au chirurgien pour une seule opération que 19 pièces au lieu normalement de 192 selon la technique antérieure, le choix étant effectué pendant l'opération. En pratique, ce nombre élevé de 192 prothèses n'est jamais mis à la disposition du chirurgien pour des raisons de coût, si bien que le résultat affiné selon l'invention n'est jamais atteint selon la technique antérieure.

Bien que l'invention ait été décrite en référence à des petites articulations de la main, celle-ci convient également pour l'ensemble des systèmes articulaires de petites tailles.

## Revendications

1. Elément prothétique (1;2) pour petites articulations comprenant une tête (4;6), une queue intra-diaphysaire (3;8) qui sont deux pièces distinctes et un moyen de stabilisation (36, 42 ; 86, 62) pour stabiliser en rotation la tête (4;6) par rapport à la queue intra-diaphysaire (3;8) à laquelle elle est liée, caractérisé en ce qu'il comprend
une tige de liaison sensiblement cylindrique (5;7) s'étendant entièrement dans la tête et la queue intra-diaphysaire et ayant des extrémités (53, 52 ; 73, 72) qui sont liées rigidement de manière démontable à la tête et la queue dans des trous (44, 37 ; 64, 87) ménagés dans la tête et la queue intra-diaphysaire.

2. Elément prothétique conforme à la revendication 1, caractérisé en ce que la tête (4;6) a une surface externe sensiblement sphérique (41;61) convexe ou concave qui est décentrée par rapport à un axe longitudinal (I-I, II-II) de la queue intra-diaphysaire (3;8).

3. Elément prothétique conforme à la revendication 1 ou 2, caractérisé en ce que le moyen de stabilisation comprend un assemblage à tenon (36;86) et rainure (42;62) complémentaires.

4. Elément prothétique conforme à l'une quelconque des revendications 1 à 3, caractérisé en ce que la queue intra-diaphysaire (3;8) a une section transversale ovale.

5. Elément prothétique conforme à l'une quelconque des revendications 1 à 4, caractérisé en ce que la queue intra-diaphysaire (3;8) est en matière plastique.

6. Elément prothétique conforme à l'une quelconque des revendications 1 à 5, caractérisé en ce que la tige de liaison (5;7) est métallique.

7. Elément prothétique conforme à l'une quelconque des revendications 1 à 6, caractérisé en ce que la tête (6) présente une surface d'articulation concave sensiblement semi-sphérique (61) limitée par une butée dorsale (612) et par deux butées latérales (611).

8. Prothèse pour petite articulation comprenant un premier élément prothétique (1) conforme à l'une quelconque des revendications 1 à 6 et un second élément prothétique (2) conforme à l'une quelconque des revendications 1 à 7, la tête (4) du premier élément présentant une surface convexe (41) dont une partie est complémentaire d'une surface concave (61) de la tête (6) du second élément.

9. Prothèse conforme à la revendication 8, dans laquelle l'une des têtes (4,6) est métallique et l'autre tête est en matière plastique.

10. Prothèse conforme à la revendication 8 ou 9, dans laquelle les tiges de liaison (5;7) des premier et second éléments (1;2) sont identiques.

## Claims

1. Prosthesis component (1;2) for small joints comprising a head (4;6), an intramedullary stem (3;8) which are two separate parts and a stabilizing means (36, 42 ; 86, 62) for rotationally stabilizing the head (4;6) relative to the intramedullary stem (3;8) to which it is connected, characterized in that it comprises
a substantially cylindrical connecting rod (5;7) extending wholly within the head and the intramedullary stem and having ends (53, 52 ; 73, 72) which are removably and rigidly connected to the head and the stem in holes (44, 37 ; 64, 87) provided in the head and the intramedullary stem.

2. Prosthesis component according to claim 1, characterized in that the head (4;6) has a substantially spherical exterior surface (41;61) convex or concave which is offset relative to a longitudinal axis (I-I, II-II) of the intramedullary stem (3;8).

3. Prosthesis component according to claim 1 or 2, characterized in that the stabilizing means comprises a complementary tenon (36;86) and groove (42;62) assembly.

4. Prosthesis component according to any one of claims 1 to 3, characterized in that the intramedullary stem (3;8) has an oval transverse cross-section.

5. Prosthesis component according to any one of claims 1 to 4, characterized in that the intramedullary stem (3;8) is made of plastic material.

6. Prosthesis component according to any one of claims 1 to 5, characterized in that the connecting rod (5;7) is made of metal.

7. Prosthesis component according to any one of claims 1 to 6, characterized in that the head (6) has a substantially hemispherical concave joint surface (61) delimited by a dorsal abutment (612) and two lateral abutments (611).

8. Prosthesis for small joint comprising a first prosthesis component (1) according to any one of claims 1 to 6 and a second prosthesis component (2) according to any one of claims 1 to 7, the head (4) of the first component having a convex surface (41), a part of which is complementary from a concave surface (61) of the head (6) of the second component.

9. Prosthesis according to claim 8, in which one of the heads (4,6) is made of metal and the other head is made of plastic material.

10. Prosthesis according to claim 8 or 9, in which the connecting rods (5;7) of the first and second components (1;2) are identical.

## Patentansprüche

1. Prothetisches Element (1; 2) für kleine Gelenke mit einem Kopf (4; 6), einem intra-diaphysären Ende (3; 8), die zwei verschiedene Stücke sind, sowie mit einem Stabilisationsmittel (36, 42; 86, 62) zur Dreh-Stabilisierung des Kopfes (4; 6) bezüglich des mit ihm verbundenen intra-diaphysären Endes (3; 8), dadurch gekennzeichnet, daß sich ein im wesentlichen zylindrischer Verbindungsstiel (5; 7) ganz in den Kopf und das intra-diaphysäre Ende erstreckt, dessen Extremitäten (53, 52; 73, 72) lösbar mit dem Kopf und dem Ende in Löchern (44, 37; 64, 87) verbunden sind, die in dem Kopf und dem intra-diaphysären Ende ausgebildet sind.

2. Prothetisches Element nach Anspruch 1, dadurch gekennzeichnet, daß der Kopf (4; 6) eine im wesentlichen kugelige konvexe oder konkave äußere Oberfläche (41; 61) aufweist, die bezüglich einer longitudinalen Achse (I-I, II-II) des intra-diaphysären Endes (3; 8) verschoben ist.

3. Prothetisches Element nach Anspruch 1 oder 2, dadurch gekennzeichnet, daß das Stabilisierungsmittel eine Nut- (42; 62) und Feder- (36; 86)verbindung aufweist.

4. Prothetisches Element nach einem der Ansprüche 1 bis 3, dadurch gekennzeichnet, daß das intra-diaphysäre Ende (3; 8) einen quer-ovalen Abschnitt aufweist.

5. Prothetisches Element nach einem der Ansprüche 1 bis 4, dadurch gekennzeichnet, daß das intra-diaphysäre Ende (3; 8) aus Kunststoff besteht.

6. Prothetisches Element nach einem der Ansprüche 1 bis 5, dadurch gekennzeichnet, daß der Verbindungsstiel (5; 7) metallisch ist.

7. Prothetisches Element nach einem der Ansprüche 1 bis 6, dadurch gekennzeichnet, daß der Kopf eine im wesentlichen halbkugelige, konkave Gelenkfläche (61) aufweist, die durch einen dorsalen Anschlag (612) und zwei laterale Anschläge (611) begrenzt ist.

8. Prothese für kleines Gelenk mit einem ersten prothetischen Element (1) nach einem der Ansprüche 1 bis 6 und einem zweiten prothetischen Element (2) nach einem der Ansprüche 1 bis 7, wobei der Kopf (4) des ersten Elementes eine konvexe Fläche (41) aufweist, von der ein Teil zu einer konkaven Fläche des Kopfes (6) des zweiten Elementes komplementär ist.

9. Prothese nach Anspruch 8, bei welcher einer der Köpfe (4, 6) metallisch ist und der andere aus Kunststoff besteht.

10. Prothese nach einem der Ansprüche 8 oder 9, bei der die Verbindungsstiele (5; 7) des ersten und des zweiten Elementes (1; 2) identisch sind.
